# EUROPÄISCHE PATENTSCHRIFT

(11) **EP 1 663 478 B1**
(45) Veröffentlichungstag und Bekanntmachung des Hinweises auf die Patenterteilung: **05.10.2016**
(21) Anmeldenummer: 04763459.7
(22) Anmeldetag: 23.07.2004
(51) Int. Cl.: B01J 19/32, B01J 19/24, C07C 45/35, C07C 51/215, C07C 51/25

(54) **VERFAHREN ZUR HERSTELLUNG VON (METH)ACROLEIN UND/ODER (METH)ACRYLS URE DURCH HETEROGEN KATALYSIERTE PARTIALOXIDATION VON C3- UND/ODER C4-VORLÄUFERVERBINDUNGEN IN EINEM REAKTOR MIT THERMOBLECHPLATTENMODULEN**
METHOD FOR THE PRODUCTION OF (METH)ACROLEIN AND/OR (METH)ACRYLIC ACID BY HETEROGENEOUSLY-CATALYSED PARTIAL OXIDATION OF C3 AND/OR C4 PRECURSOR COMPOUNDS IN A REACTOR WITH HEAT-TRANSFER PLATE MODULES
PROCEDE DE PRODUCTION D'ALDEHYDE (METH)ACRYLIQUE ET/OU D'ACIDE (METH)ACRYLIQUE, PAR OXYDATION PARTIELLE EN CATALYSE HETEROGENE DE COMPOSES PRECURSEURS COMPORTANT 3 ET/OU 4 ATOMES DE CARBONE, DANS UN REACTEUR COMPRENANT DES MODULES CONSTITUES DE PLAQUES DE TOLE THERMIQUES

(30) Priorität: 24.07.2003 DE 10333867; 24.07.2003 US 489510 P; 07.04.2004 DE 102004017150
(43) Veröffentlichungstag der Anmeldung: 07.06.2006
(73) Patentinhaber: BASF SE, 67056 Ludwigshafen am Rhein (DE)
(72) Erfinder: OLBERT, Gerhard, 69221 Dossenheim (DE); HECHLER, Claus, 67063 Ludwigshafen (DE)
(86) Internationale Anmeldenummer: PCT/EP2004/008289
(87) Internationale Veröffentlichungsnummer: WO 2005/009609

(56) Entgegenhaltungen:
- EP-A- 0 867 220
- EP-A- 0 995 491
- EP-A- 1 147 807
- EP-A- 1 234 612
- DE-A- 10 031 347
- DE-A- 19 952 964
- US-A1- 2002 146 359
- US-B1- 6 419 892

## Beschreibung

Die Erfindung betrifft ein Verfahren zur Herstellung von (Meth)acrolein und/oder (Meth)acrylsäure durch Partialoxidation in der Gasphase von C3- und/oder C4-Vorläuferverbindungen in Gegenwart eines heterogenen partikelförmigen Katalysators in 1, 2 oder 3 Reaktionsstufen in einem Reaktor mit Thermoblechplattenmodulen.

Die abgekürzte Schreibweise (Meth)acrolein bezeichnet in bekannter Weise Acrolein und/oder Methacrolein. Analog wird die abgekürzte Schreibweise (Meth)acrylsäure für Acrylsäure und/oder Methacrylsäure verwendet.

Die Partialoxidation von C3- und/oder C4-Vorläuferverbindungen, wie Propylen, Propan, iso-Buten, iso-Butan, iso-Butanol, Methylether des i-Butanols, Acrolein oder Methacrolein (d.h. insbesondere von 3 oder 4 Kohlenstoffatomen enthaltenden Kohlenwasserstoffen) in der Gasphase wird bekanntermaßen in Gegenwart von heterogenen partikelförmigen Katalysatoren durchgeführt. Diese Umsetzungen sind stark exotherm, und werden bislang im großtechnischen Maßstab überwiegend in Rohrbündelreaktoren durchgeführt, mit Kontaktrohren, in denen der heterogene partikelförmige Katalysator eingebracht ist und durch die das fluide Reaktionsgemisch geleitet wird und wobei die freiwerdende Reaktionswärme indirekt, über einen Wärmeträger abgeführt wird, der im Zwischenraum zwischen den Kontaktrohren zirkuliert. Als Wärmeträger wird häufig eine Salzschmelze eingesetzt.

Derartige Verfahren sind beispielsweise in DE-A 19 62 431, DE-A 29 43 707 oder DE-A 199 52 964 beschrieben.

Die Reaktion kann ausgehend von einem Alkan in einer Reaktionsstufe zur Säure oder in einer ersten Stufe zum Aldehyd und in einer zweiten Stufe zur Säure durchgeführt werden. Eine alternative Durchführung kann in einer ersten von drei Stufen vom Alkan zum Olefin, in einer zweiten vom Olefin zum Aldehyd und in einer dritten vom Aldehyd zur Säure gehen. Ausgehend vom Olefin kann wiederum in zwei Stufen zunächst zum Aldehyd und dann zur Säure oxidiert werden oder auch in einer Stufe vom Olefin zur Säure oxidiert werden. Die Herstellung der Säure kann auch in einer Stufe ausgehend vom jeweiligen Aldehyd erfolgen. Als Aldehyd wird hierbei (Meth)acrolein verstanden und als Säure (Meth)acrylsäure.

Alternativ ist es auch möglich, die Reaktionswärme über einen Wärmeträger abzuführen, der durch plattenförmige Wärmeüberträger geleitet wird. Für plattenförmige Wärmeüberträger werden die Begriffe Wärmetauscherplatten, Wärmeübertragerplatten, Thermobleche, Thermoplatten oder Thermoblechplatten weitgehend synonym verwendet.

Wärmeübertragerplatten werden überwiegend als flächenförmige Gebilde definiert, die einen mit Zu- und Abführleitungen versehenen Innenraum mit geringer Dicke im Verhältnis zur Fläche aufweisen. Sie werden in der Regel aus Blechen, häufig aus Stahlblechen, hergestellt. Je nach Anwendungsfall, insbesondere den Eigenschaften des Reaktionsmediums sowie des Wärmeträgers, können jedoch spezielle, insbesondere korrosionsfeste, aber auch beschichtete Werkstoffe zum Einsatz kommen. Die Zu- bzw. Abführeinrichtungen für die Wärmeträger sind in der Regel an einander entgegengesetzten Enden der Wärmetauscherplatten angeordnet. Als Wärmeträger kommen häufig Wasser, aber auch Diphyl^{®} (Gemisch aus 70 bis 75 Gew.-% Diphenylether und 25 bis 30 Gew.-% Diphenyl) zum Einsatz, welche auch teilweise in einem Siedevorgang verdampfen; es ist auch der Einsatz anderer organischer Wärmeträger mit niedrigem Dampfdruck und auch ionischer Flüssigkeiten möglich.
Die Verwendung ionischer Flüssigkeiten als Wärmeträger ist in der nicht vorveröffentlichten deutschen Patentanmeldung 103 16 418.9 beschrieben. Bevorzugt sind ionische Flüssigkeiten, die ein Sulfat-, Phosphat-, Borat- oder Silikatanion enthalten. Besonders geeignet sind auch ionische Flüssigkeiten, die ein einwertiges Metall-Kation, insbesondere ein Alkalimetall-Kation, sowie ein weiteres Kation, insbesondere ein Imidazolium-Kation, enthalten. Vorteilhaft sind auch ionische Flüssigkeiten, die als Kation ein Imidazolium-, Pyridinium- oder Phosphonium-Kation enthalten.

Der Begriff Thermobleche oder Thermoblechplatten wird insbesondere für Wärmeübertragerplatten verwendet, deren einzelne, meistens zwei, Bleche durch Punkt- und/oder Rollschweißungen miteinander verbunden und häufig unter Verwendung hydraulischen Drucks plastisch unter Kissenbildung ausgeformt sind.

Der Begriff Thermoblechplatten wird vorliegend im Sinne der obigen Definition verwendet.

Reaktoren zur Durchführung von Partialoxidationen unter Verwendung von Thermoblechen sind beispielsweise aus DE-A 199 52 964 bekannt. Beschrieben ist die Anordnung eines Katalysators zur Durchführung von Partialoxidationen in einer Schüttung um Wärmeübertragerplatten in einem Reaktor. Das Reaktionsgemisch wird an einem Reaktorende dem Reaktorinnenraum zwischen den Wärmeübertragerplatten zugeführt und am entgegengesetzten Ende abgeführt und durchströmt somit den Zwischenraum zwischen den Wärmeübertragerplatten. Dadurch findet eine ständige Quervermischung des Reaktionsgemisches mit der Folge einer hohen Homogenität desselben statt und es wird bei vorgegebenem Umsatz eine wesentlich bessere Selektivität gegenüber der Durchführung der Reaktion in einem Rohrbündelreaktor erreicht.

Die DE-C 197 54 185 beschreibt einen weiteren Reaktor mit indirekter Wärmeabführung über ein Kühlmedium, das durch Wärmeübertragerplatten strömt, wobei die Wärmeübertragerplatten als Thermobleche ausgebildet sind, die aus zumindest zwei Blechplatten aus Stahl bestehen, die an vorgegebenen Punkten unter Bildung von Strömungskanälen zusammengefügt sind.

Eine vorteilhafte Weiterbildung hiervon ist in DE-A 198 48 208 beschrieben, wonach Wärmeübertragerplatten, die als von einem Kühlmedium durchströmte Thermobleche ausgebildet sind, zu Plattenpaketen mit beispielsweise rechteckigem oder quadratischem Querschnitt zusammengefasst sind und die Plattenpakete eine so genannte Einhausung aufweisen. Das eingehauste Plattenpaket ist umfangseitig anpassungsfrei und folglich mit vorgegebenen Abständen zu der Innenwand des zylindrischen Reaktorbehälters eingesetzt. Die Freiflächen zwischen dem Plattenwärmeüberträger bzw. seiner Einhausung und der Behälterinnenwand sind im oberen und unteren Bereich der Einhausung mit Leitblechen abgedeckt, um den Bypass von Reaktionsmedium um die mit Katalysator gefüllten Kammern zu vermeiden.

Ein weiterer Reaktor mit Einrichtungen zur Abführung der Reaktionswärme in Form von Plattenwärmeübertragern ist in WO-A 01/85331 beschrieben. Der Reaktor von überwiegend zylindrischer Form enthält ein zusammenhängendes Katalysatorbett, in das ein Plattenwärmeübertrager eingebettet ist.

Die großtechnische Herstellung von (Meth)acrolein und/oder (Meth)acrylsäure durch Partialoxidation von C3- und/oder C4-Vorläuferverbindungen erfolgt in Gegenwart eines geeigneten heterogenen partikelförmigen Katalysators. Um die Durchströmung der Katalysatorschüttung mit dem Prozeßgas zu bewirken, ist ein Druckgefälle zwischen Gaseintritt und -austritt erforderlich. Dieses Druckgefälle kann z.B. durch einen Überdruck am Eintritt aber auch durch Unterdruck am Austritt oder einer Kombination dieser beiden Maßnahmen aufgeprägt werden. Bei den technisch eingesetzten Katalysatoren, die eine wirtschaftliche Produktherstellung ermöglichen, liegt typisch ein Druckgefälle über die Katalysatorschüttung von 0,2 bis zu 0,6 bar vor, und relativ zur Umgebung kann der Druckunterschied durchaus auch 1 bar und gegebenenfalls mehr betragen.

Bei Rohrreaktoren wird dieser Druckunterschied zur Umgebung durch eine zylindrische Ausführung der druckbeaufschlagten Wände getragen. Ausgedehnte Untersuchungen an Reaktoren mit Thermoblechen haben ergeben, dass sich insbesondere Probleme durch Deformationen aufgrund einseitiger hoher Belastung der Thermobleche bei zu großem Druckunterschied zwischen dem Reaktionsgemisch und der äußeren Umgebung ergeben, sowie mechanische Stabilitätsprobleme durch Verformung unter starker thermischer Beanspruchung zeigen. Diese Probleme können auftreten, wenn das Reaktionsgemisch unter Überdruck steht, aber auch, wenn die Reaktion bei Unterdruck betrieben wird.

Die EP-A 0 995 491 beschreibt ebenfalls einen Reaktor mit Thermoblechplatten (Plattenwärmetauschern) wie er im erfindungsgemäßen Verfahren zur Herstellung von (Meth)acrolein und/oder (Meth)acrylsäure eingesetzt wird, jedoch sind hierbei die Freiflächen zwischen dem Plattenwärmetauscher bzw. seiner Einhausung und der Behälterinnenwand im oberen und unteren Bereich der Einhausung mittels Leitblechen lediglich abgedeckt.

Der Reaktor aus EP 1 147 807 ist in Modulbauweise aufgebaut, unter Ausbildung von Einzelreaktoren im Reaktorbehälter, mit Trennwänden zwischen den Einzelreaktoren, wobei die Trennwände mit der die Einzelreaktoren umgebenden Behälteraußenwand kraftschlüssig verbunden sind und Zuganker bilden. Die EP 1 147 807 gibt zwar einen Hinweis auf die Notwendigkeit, eine stabile Bauweise für einen Reaktor mit Thermoblechmodulen zu gewährleisten, weist jedoch zur Lösung dieses Problems in eine andere Richtung:

Aufgabe der Erfindung war es, einen Reaktor mit Abführung der Reaktionswärme über einen Wärmeträger, der Thermoblechplatten durchströmt, zur Verfügung zu stellen, der für die großtechnische Herstellung von (Meth)acrolein und/oder (Meth)acrylsäure durch Partialoxidation von C3/C4-Vorläuferverbindungen in Gegenwart eines heterogenen partikelförmigen Katalysators wirtschaftlich und störungsfrei, insbesondere unter Vermeidung der vorstehend dargelegten Probleme, betrieben werden kann. Durch die Erfindung soll die geometrische Stabilität der Thermoblechplattenmodule, besonders der zur Aufnahme des Katalysators bestimmten Spalte, speziell bei Durchführung der Reaktion zu (Meth)acrolein und/oder (Meth)acrylsäure gewährleistet sein.

Die Lösung besteht in einem Verfahren entsprechend der Definition in Anspruch 1.

Erfindungsgemäß wird somit eine Partialoxidation von C3- und/oder C4-Vorläuferverbindungen zu (Meth)acrolein und/oder (Meth)acrylsäure in einem Reaktor durchgeführt, der Thermoblechplattenmodule umfasst, die wiederum aus Thermoblechplatten gebildet sind, durch die ein Wärmeträger strömt, der die Reaktionswärme aufnimmt und hierbei zumindest teilweise verdampft. Die Thermoblechplattenmodule sind quaderförmig ausgebildet und druckentlastend in einer dieselben vollständig umgebenden überwiegend zylinderförmigen Hülle eingebracht.

Die Thermoblechplattenmodule sind aus jeweils zwei oder mehreren rechteckigen, parallel zueinander unter Freilassung jeweils eines Spaltes angeordneten Thermoblechplatten gebildet.

Die Thermoblechplatten sind aus korrosionsfreien Werkstoffen, vorzugsweise aus Edelstahl, beispielsweise mit der Werkstoffnummer 1.4541 bzw. 1.4404, 1.4571 bzw. 1.4406, 1.4539 aber auch 1.4547 oder aus anderen legierten Stählen, gefertigt.

Die Materialstärke der hierfür eingesetzten Bleche kann zwischen 1 und 4 mm, 1,5 und 3 mm, aber auch zwischen 2 und 2,5 mm, oder zu 2,5 mm gewählt werden.

In der Regel werden zwei rechteckige Bleche an ihren Längs- und Stirnseiten zu einer Thermoblechplatte verbunden, wobei eine Rollnaht oder seitliches Zuschweißen oder eine Kombination von beidem möglich ist, so dass der Raum, in dem sich später der Wärmeträger befindet, allseitig dicht ist. Vorteilhaft wird der Rand der Thermoblechplatten an oder schon in der seitlichen Rollnaht der Längskante abgetrennt, damit der schlecht oder nicht gekühlte Randbereich, in dem meist auch Katalysator eingebracht ist, eine möglichst geringe geometrische Ausdehnung hat.

Über die Rechteckfläche verteilt werden die Bleche miteinander durch Punktschweißung verbunden. Auch eine zumindest teilweise Verbindung durch gerade oder auch gebogene und auch kreisförmige Rollnähte ist möglich. Auch die Unterteilung des vom Wärmeträger durchströmten Volumens in mehrere getrennte Bereiche durch zusätzliche Rollnähte ist möglich.

Eine Möglichkeit der Anordnung der Schweißpunkte auf den Thermoblechplatten ist in Reihen mit äquidistanten Punktabständen von 30 bis 80 mm oder auch 35 bis 70 mm, wobei auch Abstände von 40 bis 60 mm möglich sind, wobei eine weitere Ausführungsform Abstände von 45 bis 50 mm und auch 46 bis 48 mm ist. Typischerweise variieren die Punktabstände fertigungsbedingt bis zu ± 1 mm und die Schweißpunkte unmittelbar benachbarter Reihen sind in Längsrichtung der Platten gesehen, jeweils um einen halben Schweißpunktabstand versetzt angeordnet. Die Reihen der Punktschweißungen in Längsrichtung der Platten können äquidistant mit Abständen von 5 bis 50 mm, aber auch von 8 bis 25 mm, wobei auch Abstände von 10 bis 20 mm und auch 12 bis 14 mm, eingesetzt werden. Weiterhin sind auch dem Anwendungsfall angepasste Paarungen der genannten Schweißpunktabstände und Reihenabstände möglich. Die Reihenabstände können in einem definierten geometrischen Zusammenhang zum Punktabstand, typisch ¼ der Punktabstände oder etwas geringer sein, so dass sich eine definiert gleichmäßige Aufweitung der Thermobleche bei der Herstellung ergibt. Vorgegebenen Schweißpunkt- und Reihenabständen ist eine entsprechend Anzahl von Schweißpunkten je m² Plattenoberfläche zugeordnet.

Die Breite der Thermoblechplatten ist im Wesentlichen fertigungstechnisch begrenzt und kann zwischen 100 und 2500 mm, oder auch zwischen 500 und 1500 mm, liegen. Die Länge der Thermoblechplatten ist abhängig von der Reaktion, insbesondere vom Temperaturprofil der Reaktion, und kann zwischen 500 und 7000 mm, oder auch zwischen 3000 und 4000 mm liegen.

Jeweils zwei oder mehrere Thermoblechplatten sind parallel und beabstandet zueinander, unter Bildung eine Thermoblechplattenmodules, angeordnet. Dadurch entstehen zwischen unmittelbar benachbarten Blechplatten schachtartige Spalte, die an den engsten Stellen des Plattenabstandes beispielsweise eine Breite zwischen 8 und 150 mm, aber auch 10 bis 100 mm aufweisen. Eine mögliche Ausführung sind auch Breiten von 12 bis 50 mm oder aber 14 bis 25 mm, wobei auch 16 bis 20 mm gewählt werden können. Es wurde auch schon ein Spaltabstand von 17 mm erprobt.

Zwischen den einzelnen Thermoblechplatten eines Thermoblechplattenmodules können, z.B. bei großflächigen Platten, zusätzlich Distanzhalter eingebaut werden, um Verformungen vorzubeugen, welche Plattenabstand oder -position verändern können. Zum Einbau dieser Distanzhalter können Teilbereiche der Bleche durch zum Beispiel kreisförmige Rollnähte vom Durchflussbereich des Wärmeträgers abgetrennt werden, um dort beispielsweise Löcher für Befestigungsschrauben der Distanzhalter in die Platten einbringen zu können.

Die Spalte können gleichen Abstand besitzen, bei Erfordernis können die Spalte aber auch unterschiedlich breit sein, wenn die Reaktion dies zulässt oder die gewünschte Reaktion es erfordert, oder apparative oder kühltechnische Vorteile erzielt werden können.

Die mit Katalysatorpartikeln gefüllten Spalte eines Thermoblechplattenmodules können gegeneinander gedichtet, z.B. dichtgeschweißt sein oder auch prozessseitig zueinander Verbindung besitzen.

Zur Einstellung des gewünschten Spaltabstandes beim Zusammenfügen der einzelnen Thermoblechplatten zu einem Modul werden die Platten in ihrer Position und im Abstand fixiert.

Die Schweißpunkte unmittelbar benachbarter Thermoblechplatten können sich gegenüberliegen oder versetzt zueinander sein.

In der Regel wird es aus fertigungstechnischen Gründen bevorzugt sein, bei der Anordnung mit zwei oder mehreren quaderförmigen Thermoblechplattenmodulen, dieselben mit jeweils gleichen Abmessungen auszubilden. Bei Anordnungen von 10 oder 14 Thermoblechplattenmodulen kann es für die Kompaktheit des Gesamtapparates vorteilhaft sein, zwei Modultypen mit unterschiedlicher Kantenlänge bzw. unterschiedlichem Kantenlängenverhältnis zu wählen.

Bevorzugt sind Anordnungen von 4, 7, 10 oder 14 Thermoblechplattenmodulen mit jeweils gleichen Abmessungen. Die in Strömungsrichtung sichtbare Projektionsfläche eines Moduls kann quadratisch sein, aber auch rechteckig mit einem Seitenverhältnis von 1,1 aber auch 1,2. Vorteilhaft sind Kombinationen von 7, 10 oder 14 Modulen mit rechteckigen Modulprojektionen, so dass der Durchmesser der äußeren zylindrischen Hülle minimiert wird. Besonders vorteilhafte geometrische Anordnungen sind erzielbar, wenn, wie oben aufgeführt, eine Anzahl von 4, 7 oder 14 Thermoblechplattenmodulen gewählt wird.

Vorteilhaft sollen hierbei die Thermoblechplattenmodule einzeln auswechselbar sein, beispielsweise bei Leckagen, Verformungen der Thermobleche oder bei Problemen, die den Katalysator betreffen.

Vorteilhaft sind die Thermoblechplattenmodule in jeweils einem druckstabilen, rechteckigen Stabilisierungskasten angeordnet.

Jedes Thermoblechplattenmodul wird vorteilhaft durch eine geeignete Führung, beispielsweise durch die rechteckigen Stabilisierungskästen, mit seitlich durchgehender Wandung oder beispielsweise durch eine Winkelkonstruktion in Position gehalten.

In einer Ausführungsform sind die rechteckigen Stabilisierungskästen benachbarter Thermoblechplattenmodule gegeneinander abgedichtet. Dadurch wird eine Bypass-Strömung des Reaktionsgemisches zwischen den einzelnen Thermoblechplattenmodulen verhindert.

Durch den Einbau von quaderförmigen Thermoblechplattenmodulen in eine überwiegend zylindrische drucktragende Hülle verbleiben am Rand zur zylindrischen Mantelwand der Hülle relativ große freie Zwischenräume, in denen eine Ablagerung, Nebenreaktionen oder eine Zersetzung des Werkproduktes stattfinden können. Eine Reinigung, Dekontamination von Produkt zum Beispiel bei der Notwendigkeit von Montagetätigkeiten ist dort nur stark erschwert möglich. Es ist daher vorteilhaft, diesen Zwischenraum vom Reaktionsraum, das heißt von den Spalten zwischen jeweils unmittelbar benachbarten Thermoblechplatten, zu separieren.

Hierzu verschließt man den Zwischenraum zwischen den Thermoblechplattenmodulen und der überwiegend zylinderförmigen Hülle am unteren Ende der Thermoblechplattenmodule mit einem Halteboden. Um eine Bypass-Strömung des Reaktionsgemisches zu verhindern, soll der Trag- oder Halteboden den Zwischenraum gasdicht verschließen.

Vorteilhaft kann der Zwischenraum zwischen den Thermoblechplattenmodulen und der überwiegend zylinderförmigen Hülle auch am oberen Ende der Thermoblechplattenmodule durch eine Blechabdeckung verschlossen werden. Hierfür ist jedoch ein gasdichter Verschluss nicht notwendig, es ist in einer Ausführungsform möglich, die Blechabdeckung mit Öffnungen auszubilden.

Die Blechabdeckung am oberen Ende des Zwischenraumes zwischen den Thermoblechplattenmodulen und der überwiegend zylinderförmigen Hülle kann vorteilhaft auch ähnlich einem Ventilboden ausgebildet sein.

Die Abströmung des zur Druckbeaufschlagung verwendeten Gases kann weiterhin mittels eines Überströmorgans, als Blende, Ventil oder kraftbelastetes (zum Beispiel mit Feder oder Gasdruck), selbstregelndes Organ ausgeführt, auch in Kombination mit einer Rückschlagsicherung, hergestellt werden. Diese Überströmorgane können auch außerhalb der zylindrischen Außenhülle angeordnet sein.

Die obere Blechabdeckung kann auf Verstrebungen sitzen, welche die rechteckigen Stabilisierungskästen, in denen die Thermoblechplattenmodule eingebracht sind, zusätzlich stabilisieren.

Der Zwischenraum zwischen den Thermoblechplattenmodulen und der vorwiegend zylinderförmigen Hülle kann vorteilhaft mit Inertstoffen ausgefüllt werden, um das freie Gasvolumen dort zu verringern und um Gaskonvektion zu verhindern, welche beispielsweise zu unkontrolliertem Wärmeabfluss führen kann.

In der zylinderförmigen Hülle sind vorteilhaft Stutzen für die Zu- und Abführung des Inertstoff-Schüttgutes vorgesehen, die in geeigneter Größe ausgeführt und Neigung angebracht sind, so dass eine staufreie Befüllung und Entleerung schwerkraftgetrieben möglich ist. Mögliche Ausführungsformen der Stutzen sind Nennweiten von 80, 100, 150 oder 200 mm.

Als Inertstoff-Schüttgut kann grundsätzlich jedes chemisch inerte und mechanisch und thermisch ausreichend stabile Material eingesetzt werden, beispielsweise expandierter Perlite und/oder expandierter Vermiculit.

Der Zwischenraum zwischen den Thermoblechplattenmodulen und der überwiegend zylinderförmigen Hülle, der mit Inertmaterial ausgefüllt sein kann, ist mit einem Gasdruck beaufschlagt.

Die Druckbeaufschlagung kann im Wesentlichen statisch sein und wird durch die druckgeregelte Zu- und Abfuhr von Stickstoff bewirkt. Als Regelsignal kann beispielsweise der Druckunterschied zwischen dem Druck im Zwischenraum zwischen den Thermoblechplattenmodulen und der überwiegend zylinderförmigen Hülle und der Druck am unteren Ende der Katalysatorschüttung in den Spalten der Thermoblechplattenmodule oder am oberen Ende desselben gewählt werden. Vorteilhaft kann das Differenzdrucksignal um einen Offsetwert korrigiert sein, bevorzugt kann ein Mittelwert, insbesondere der arithmetische Mittelwert des Druckes über die Höhe der Katalysatorschüttung als Regelsignal gewählt werden.

Zur Druckbeaufschlagung können in der überwiegend zylinderförmigen Hülle entsprechende Stutzen und/oder eine interne Ringleitung mit kleinen Bohrungen vorgesehen sein, die vorzugsweise nach unten gerichtet sind.

Alternativ wird die Druckbeaufschlagung unter kontinuierlicher Durchströmung des Zwischenraumes mit einem prozessinerten oder prozessintrinsischen Gas, insbesondere mit Stickstoff oder mit Kreisgas bewirkt.

Das zur Druckbeaufschlagung verwendete Gas wird vorteilhaft mit dem fluiden Reaktionsgemisch an dessen Austritt aus den Thermoblechplattenmodulen vereinigt, in der Regel noch innerhalb der überwiegend zylinderförmigen Hülle des Reaktors. Die Ausströmstellen des zur Druckbeaufschlagung verwendeten Gases sind vorteilhaft in Strömungstotzonen des fluiden Reaktionsgemisches gelegt, um dieselben zu spülen.

Der Volumenstrom des zur Druckbeaufschlagung verwendeten Gases wird in der Regel signifikant kleiner sein als der Volumenstrom des fluiden Reaktionsgemisches und wird vorteilhaft so gewählt werden, dass er für die Reaktion prozesstechnisch unschädlich ist.

Die Thermoblechplattenmodule sollen vorteilhaft jeweils einzeln austauschbar sein, um, wie vorstehend bereits ausgeführt, bei auftretenden Problemen, beispielsweise Leckagen, Verformungen der Thermobleche oder Problemen mit dem Katalysator, gezielt Abhilfe schaffen zu können. Hierfür ist es vorteilhaft, die Thermoblechplattenmodule mit etwas Spiel gegenüber der Wandung der rechteckigen Stabilisierungskästen auszubilden.

Da die Thermoblechplattenmodule somit bei dieser vorteilhaften Ausführung nichtdichtend in den rechteckigen Stabilisierungskästen sitzen, kann es zu Bypass-Strömungen des Reaktionsmediums kommen. Um dies zu vermeiden, werden die Undichtigkeiten zwischen den Thermoblechplattenmodulen und den rechteckigen Stabilisierungskästen in geeigneter Weise abgedichtet, beispielsweise mit auf der Außenseite der Thermoblechplattenmodule angebrachten Metallblechstreifen, die sich beim Einschieben in den rechteckigen Stabilisierungskasten an die Wand desselben drücken. Alternativ sind gasdichte Blechabdeckungen und -verbindungen, beispielsweise in Form von Schweißlippen-Dichtungen möglich.

Nach dem Einschieben der Thermoblechplattenmodule in die rechteckigen Stabilisierungskästen können dieselben gegen den Halteboden abgedichtet werden, der den Zwischenraum zwischen den Thermoblechplattenmodulen und der überwiegend zylinderförmigen Hülle am unteren Ende der Thermoblechplattenmodule verschließt. Hierfür ist grundsätzlich jede bekannte Dichtungsmöglichkeit einsetzbar. Es kann sich um herkömmliche Dichtungen handeln, die beispielsweise zusätzlich verschraubt sind.

Es ist auch möglich, die Abdichtung durch Schweißlippen zu bewirken, beispielsweise durch eine Variante, bei der eine Schweißlippe am Halteboden befestigt ist und eine zweite Schweißlippe am Außenrand des Thermoblechplattenmodules oder des rechteckigen Stabilisierungskastens. Beide Schweißlippen sind so gestaltet, dass sie geometrisch zusammenpassen und zusammengeschweißt werden können. Zum Austausch des Thermoblechplattenmodules wird die Schweißnaht aufgetrennt und bei Bedarf erneuert.

Die Thermoblechplattenmodule können durch eine Einrichtung von oben mit den rechteckigen Stabilisierungskästen verspannt werden. Durch genügend Spanndruck von oben wird eine ausreichende Flächenpressung auf der Dichtung erreicht und die Thermoblechplattenmodule werden vorteilhaft fixiert.

Die rechteckigen Stabilisierungskästen müssen nicht zwingend gegeneinander abgedichtet sein, solange eine unzulässige Bypassströmung an den Spalten vorbei verhindert wird. Es ist auch möglich, die rechteckigen Stabilisierungskästen untereinander mit kleinen Bohrungen zu verbinden, durch die Inertgas aus dem Zwischenraum zwischen Thermoblechplattenmodulen und der überwiegend zylinderförmigen Hülle einströmen kann, wodurch im Raum zwischen dem Thermoblechplattenmodul und dem rechteckigen Stabilisierungskasten Reaktionen verhindert werden.

Die Thermoblechplattenmodule können zusätzlich auch an der Außenseite Führungs- und Ausrichtungselemente aufweisen. Es ist beispielsweise möglich, an den Ecken derselben Eckwinkel jeglicher Form und an ihrer Seite konische Blechstreifen vorzusehen. Weiterhin ist es von Vorteil an den Modulen Anschlagvorrichtungen oder Anschlaghilfsmittel wie Ösen, Laschen oder Gewindebohrungen anzubringen, um ein einfaches Einsetzen mittels eines Hebezeugs oder z. B. eines Krans zu ermöglichen. Zum Einkranen der Thermoblechplattenmodule können diese auch an Zugankern gehalten werden, welche vertikal durch die zunächst leeren Spalte bis zur Unterkante der Platten reichen und dort mit einem Querträger zur Lastaufnahme verbunden sind.

In einer besonderen Ausführungsform kann die äußerste Thermoblechplatte eines Thermoblechplattenmodules an der äußeren Seite derselben aus einem gegenüber den übrigen zur Herstellung der Thermoblechplatten eingesetzten Blechen dickeren und somit stabileren Blech gebildet sein.

Zur Kompensierung der thermischen Ausdehnung sind im oder am Halteboden, welcher den Zwischenraum zwischen den Thermoblechplattenmodulen und der überwiegend zylinderförmigen Hülle am unteren Ende der Thermoblechplattenmodule verschließt, vorteilhaft insbesondere ringförmige Kompensatoren vorgesehen. Besonders geeignet ist eine ringförmige Kompensation mit einem in Richtung senkrecht zur Ebene des Blechbodens betrachteten annähernd z-förmigen Profil. Gleichmaßen möglich sind jedoch auch herkömmliche, wellenförmige Kompensatoren.

Weiterhin sind bevorzugt auch in oder an der Blechabdeckung am oberen Ende des Zwischenraums zwischen Thermoblechplattenmodulen und überwiegend zylinderförmiger Hülle Kompensatoren für die axiale und/oder radiale Ausdehnung vorgesehen.

Jedes Thermoblechplattenmodul wird durch eine oder mehrere Verteileinrichtungen mit dem Wärmeträger versorgt. Dieser wird nach Durchströmen des Innenraums in den einzelnen Thermoblechplatten am anderen Ende des Thermoblechplattenmodules über eine oder mehrere Sammeleinrichtungen abgezogen. Da erfindungsgemäß ein Wärmeträger eingesetzt wird, der freiwerdende Reaktionswärme aufnimmt und hierbei zumindest teilweise verdampft, ist es zur Anpassung der Strömungsgeschwindigkeiten besonders vorteilhaft, pro Thermoblechplattenmodul jeweils eine Verteileinrichtung, jedoch zwei Sammeleinrichtungen vorzusehen.

Die Verteil- und Sammeleinrichtungen sind bevorzugt in der Weise ausgebildet, dass sie jeweils eine Kompensation für die Aufnahme der thermischen Ausdehnung der Thermoblechplattenmodule relativ zur umgebenden überwiegend zylinderförmigen Hülle aufweisen. Hier ist eine Kompensation beispielsweise durch eine geschwungene Rohrleitungsführung möglich.

Möglich ist es, zur Aufnahme der thermischen Ausdehnung der Thermoblechplattenmodule relativ zur umgebenden überwiegend zylinderförmigen Hülle eine geeignete bogenförmige oder Z- bzw. Omega-förmige geometrische Ausgestaltung der Verrohrung der Verteil- und Sammeleinrichtungen für den die Thermoblechplatten durchströmenden Wärmeträger zu gewährleisten. In einer weiteren Ausführungsform kann diese Kompensation durch axiale oder laterale Kompensatoren erfolgen, wobei eine gegebenenfalls erforderliche Rohrabstützung an einem inneren Tragwerk erfolgen kann.

Die Sammelrohre an den Thermoblechplatten für die Zufuhr und Verteilung sowie Sammlung und Abfuhr des Wärmeträgers werden besonders bevorzugt mit einer sogenannten Schlitzbodeneinschweißung wie folgt ausgeführt: Die einzelnen Thermoblechplatten eines Moduls werden zunächst mit einem zum Innenraum der Thermoblechplatten hin gewölbten rinnenförmigen Blech, das einen annähernd halbkreisförmigen Querschnitt sowie Öffnungen oder Schlitze für den Austritt des Wärmeträgers aufweist, verbunden. In diesem Zustand der Fertigung ist es möglich die Schlitzbodeneinschweißungen sowohl in einer repräsentativen Stichprobe aber auch in vollem Umfang z.B. durch Röntgenstrahlen auf Freiheit von Fertigungsfehlern zu prüfen. Anschließend wird dieses erste, annähernd rinnenförmige Blech, mit einem zweiten analog geformten Blech, jedoch mit entgegengesetzter Wölbung und ohne Öffnungen oder Schlitze, an den beiden Längsseiten verbunden, insbesondere durch Längsnahtschweißung, wobei ein rohrförmiges Bauteil mit annähernd kreisförmigem Querschnitt entsteht. Die beiden Enden dieses rohrförmigen Bauteiles werden durch Deckel verschlossen, die gegebenenfalls durch einen innenliegenden Zuganker verstärkt sein können.

In einer weiteren Ausführungsform ist auch die direkte Anschweißung von Rohrteilen mit eher kleiner Nennweite von z.B. 4 bis 30 mm an die Thermoblechplatten häufig an den Blechrändern zur Zu- bzw. Abfuhr des Wärmeträgers möglich.

Die Spalte zwischen den einzelnen Thermoblechplatten jedes Thermoblechplattenmodules dienen der Aufnahme des heterogenen partikelförmigen Katalysators. Auf die Oberseite der Katalysatorschüttung oder auf dem oberen Ende der Thermoblechplattenmodule kann eine Abdeckung, z.B. ein Lochblech, aufgebracht werden, die bei Anströmung der Schüttung von unten mit hoher Strömungsgeschwindigkeit ein Lockern, Fluidisieren oder einen Austrag von Katalysator vermeidet.

Um ein Abfließen der Katalysatorpartikel aus den Spalten unter Einwirkung der Schwerkraft auszuschließen, müssen am unteren Ende derselben geeignete Katalysatorhalteroste vorgesehen sein. Dies kann z.B. mit Loch- oder Gitterblechen erfolgen, besonders vorteilhaft können hierfür so genannte Kantenspaltsiebe eingesetzt werden, die eine gute Rückhaltung des Katalysators bei gleichzeitig hoher Formstabilität und geringem Druckverlust für das durchströmende Reaktionsmedium gewährleisten.

Die Katalysatorhalteroste können beispielsweise schwenkbar eingebaut sein.

Besonders vorteilhaft ist es, wenn die Verteileinrichtungen für den Wärmeträger zu den Thermoblechplatten so eingebaut werden, dass die seitlichen Abstände von den Verteileinrichtungen zum Rand des Thermoblechplattenpaketes gleich sind, so dass nur ein einziger Typ von Katalysatorhalterost erforderlich ist. Vorteilhaft werden pro Thermoblechplattenmodul jeweils zwei Katalysatorhalterroste vorgesehen, das heißt beidseitig der Verteileinrichtung für den Wärmeträger.

Die Katalysatorhalteroste sind vorteilhaft so zu dimensionieren, dass sie über die Mannlöcher in der überwiegend zylinderförmigen Hülle ein- und ausgebaut werden können. Häufig haben die Mannlöcher einen Innendurchmesser von 700 mm. Entsprechend ist eine Kantenlänge für die Katalysatorauflageroste von 650 mm bevorzugt.

In einer weiteren Ausführungsform ist es möglich diese Halteroste in kleinere Einheiten weiter zu unterteilen, aber auch jeden Spalt oder jede Spalthälfte einzeln zu verschließen, so dass er auch getrennt entleert werden kann.

Alternativ ist es auch möglich, die Thermoblechplattenmodule vor dem Einbau derselben in den Reaktor, d.h. außerhalb des Reaktors, mit Katalysator zu befüllen.

Die die Thermoblechplattenmodule umgebende Hülle wurde vorstehend als überwiegend zylinderförmig bezeichnet. Darunter wird verstanden, dass sie einen Zylindermantel mit kreisförmigem Querschnitt aufweist, der an beiden Enden jeweils durch eine Haube verschlossen ist.

Die überwiegend zylinderförmige Hülle wird in der Regel vertikal aufgestellt.

Das fluide Reaktionsmedium wird über die eine Haube, häufig über die untere Haube, in den Reaktorinnenraum geleitet, durchströmt die mit dem heterogenen partikelförmigen Katalysator gefüllten Spalte zwischen den einzelnen Thermoblechplatten und wird am anderen Ende des Reaktors, über die andere, häufig die obere, Haube gezogen.

Die Hauben sind bevorzugt aus Edelstahl gefertigt oder mit Edelstahl-plattiert.

Die Hauben können fest verschweißt oder trennbar, beispielsweise über eine Flanschverbindung, mit dem Zylindermantel der Hülle verbunden sein. Die Flanschverbindung kann mittels eines Hydrauliksystems absenkbar ausgestaltet sein.

Die Hauben sind vorteilhaft über ein oder mehrere Mannlöcher, die in der Regel einen Durchmesser von 700 mm aufweisen, am Umfang begehbar. Hierzu ist ein erhöhter zylindrischer Schuss von Vorteil, der, wie auch die Haube, beispielsweise aus Edelstahl gefertigt oder mit Edelstahl-plattiert ist.

Über die Mannlöcher in den Hauben besteht Zugang zur Oberseite der Module, so dass der Katalysator einfach in die Spalte zwischen den Thermoblechplatten eingebracht werden kann, und zur Unterseite der Module, so dass die Halteroste leicht montiert und demontiert werden können.

Zum Ausbau des Katalysators können in der unteren Haube zusätzlich Vorrichtungen zum Haltern von Hilfsmitteln und zum Auffangen des Katalysators, die bereits beim Betrieb eingebaut sein können, sowie ein oder mehrere Stutzen zum Ablassen des Katalysators vorgesehen werden.

Als Werkstoff für den Zwischenraum zwischen den Thermoblechplattenmodulen und der Innenwand der überwiegend zylinderförmigen Hülle verschließenden Halteboden wie auch für die rechteckigen Stabilisierungskästen für die Thermoblechplattenmodule kann Kohlenstoffstahl verwendet werden. Alternativ ist es möglich, auch hierfür Edelstahl einzusetzen.

In einer oder beiden Hauben sind vorteilhaft Stutzen montiert, durch die Multithermoelemente in die einzelnen Thermoblechplattenmodule eingeführt werden können. Weiterhin können dort Stutzen für weitere Feldgeräte und Prozessmesseinrichtungen angebracht sein.

Bevorzugt sind im zylindrischen Mantel der überwiegend zylinderförmigen Hülle ein oder mehrere Kompensatoren zur Aufnahme vorzugsweise der axialen thermischen Ausdehnung vorgesehen.

Gegenstand der Erfindung ist die Verwendung eines Reaktors zur Durchführung von Partialoxidationen eines fluiden Reaktionsgemisches, wobei die Reaktionswärme durch einen die Thermoblechplatten durchströmenden Wärmeträger abgeführt wird, der hierbei zumindest teilweise verdampft.

Der Reaktor wird bei den stark exothermen Reaktionen zum Beispiel in der Weise betrieben, dass man das fluide Reaktionsgemisch über die untere Haube zuführt und aus dem Reaktor über die obere Haube abzieht.

Da das Wärmeträgermedium, das insbesondere durch Siedekühlung die Reaktionswärme abführt, von unten in die Thermobleche zugeführt wird, steht somit bei der Zuführung des Reaktionsgemisches von unten, das heißt bei Gleichstromführung von Reaktionsgemisch und Wärmeträger, stets genügend Wärmeträger zur Kühlung zur Verfügung.

Darüber hinaus muss sowohl konstruktiv wie auch von der Strömungsführung des Reaktionsmediums und der Betriebsweise gewährleistet sein, dass weder das Reaktionsmedium vor Erreichen der aktiven Katalysatorzone durch unterkühlten Wärmeträger zu stark abgekühlt wird, noch dass der Wärmeträger in unzulässig hohem Maße vorverdampft.

Als Wärmeträgermedium kann Speisewasser eingesetzt werden, wie es typischerweise in Kraftwerken zur Dampferzeugung genutzt wird und dem Stand der Technik (Technische Regeln für Dampfkessel (TRD) 611 vom 15. Oktober 1996 in BArbBI. 12/1996 s. 84, zuletzt geändert am 25. Juni 2001 in BArbBI. 8/2001 S. 108) entspricht. Typische Parameter des Speisewassers können sein: Leitfähigkeit kleiner 0,4 oder kleiner 0,2 Mikrosiemens/cm, Kalzium- und Magnesiumhärte kleiner 0,0005 Millimol je Liter oder unter der Nachweisgrenze, Natrium kleiner 5 Mikrogramm je Liter, Siliziumdioxid kleiner 20 Mikrogramm je Liter, Eisen kleiner 50 Mikrogramm je Liter und Sauerstoff kleiner 20 Mikrogramm je Liter und ein Gesamtgehalt an gelöstem Kohlenstoff von weniger als 0,2 Milligramm je Liter. Darüber hinaus sollte das Speisewasser arm oder frei an Halogen insbesondere Chlor sein. Es ist auch möglich, das Speisewasser z.B. durch Zugabe von Hilfsstoffen wie Hydrazin, Ammoniak gezielt zu konditionieren, insbesondere alkalisch zu stellen, weiterhin können dem Speisewasser Korrosionsinhibitoren zugesetzt werden.

Die obere Haube, durch die das Reaktionsmedium bei der oben beschriebenen bevorzugten Prozessführung den Reaktor verlässt, kann aus Kohlenstoffstahl bestehen.

Um den Zugang zu den Thermoblechplattenmodulen zwecks Reparatur oder Austausch zu gewährleisten, muss die obere Haube ebenfalls entfernt werden können. Wenn keine Flanschverbindung vorhanden ist, kann die obere Haube abgetrennt und nach Modulmontage wieder angeschweißt werden.

Eine Integrierung des aus den Thermoblechplatten abgezogenen Dampfes in unterschiedliche Dampfschienen ist möglich.

Der Reaktor kann optional an zwei Dampfschienen angeschlossen werden, davon eine mit höherem Druck, welche für die Aufheizung des Reaktors auf Betriebstemperatur genutzt werden kann.

Vorteilhaft ist der Betrieb an nur einer Dampfschiene.

Der Reaktor kann vorzugsweise mit Naturumlauf des Kühlmediums Wasser betrieben werden, wobei das Verhältnis von Speisewasser zu Dampf in der Regel 3 bis 12, vorzugsweise 5 bis 10, beträgt.

Möglich ist auch der Betrieb mit Zwangsumlauf, wobei dann eine breitere Lastvariation der Kühlung möglich ist. Das Speisewasser wird hierzu mit einem höheren Druck als im Kühlsystem vorhanden beispielsweise mittels einer Pumpe zugeführt.

Es kann eine Speisewasserumlaufgeschwindigkeit in den Verteileinrichtungen zwischen 0,5 und 3,0 m/s, oder auch von 1,0 bis 2,0 m/s und eine Wasserumlaufzahl zwischen 3 und 12 eingestellt werden. Die Strömungsgeschwindigkeit der Zweiphasenströmung (Dampf/Wasser) in den Sammeleinrichtungen kann zwischen 0,5 und 15 m/s, oder auch zwischen 2,0 und 6,0 m/s liegen.

Besonders bevorzugt führt man die Aufheizung der Thermoblechplattenmodule zur Inbetriebnahme des Reaktors aus demselben Wärmeträgernetz durch, in das beim Reaktionsbetrieb die Wärme durch zumindest teilverdampftes Wärmeträgermedium abgeführt wird.

Durch die Regelung des Dampfdruckes im Kühlsystem ist eine präzise Einstellung der Kühltemperatur möglich. Die Thermoblechplatten können erfahrungsgemäß bis zu einem Druck von etwa 80 bar im Kühlmittel betrieben werden. Der Reaktor für das erfindungsgemäße Verfahren ermöglicht die Direktdampferzeugung bei Druckniveaus bis 80 bar.

Der Reaktor kann zur Durchführung der erfindungsgemäßen Partialoxidationen in großtechnischem Maßstab eingesetzt werden.

Gegenüber der Befüllung einer großen Anzahl, häufig fünfstelligen Zahl von Kontaktrohren, ist die Bereitstellung des Katalysators und die Befüllung einer bei gleicher Produktkapazität zwei oder dreistelligen Anzahl der Spalte zwischen den Thermoblechen mit Katalysator mit deutlich geringerem Aufwand verbunden.

Durch die modulare Ausgestaltung kann der Reaktor flexibel an die erforderliche Kapazität angepasst werden. Es kann eine geringere Anzahl von Thermoblechplattenmodulen im Vergleich zur durch die relative Geometrie der Hülle und der Thermoblechplattenmodule begrenzten maximal möglichen Anzahl eingebaut oder betrieben werden. Auch ist es möglich bei Bedarf oder Erfordernis einzelne Module gegen Prozessgasdurchfluss abzuschotten und die Reaktion bei gleichen äußeren Bedingungen mit verringerter Kapazität zu betreiben.

Es ist möglich, den Reaktor für das erfindungsgemäße Verfahren in Einzelteilen anzuliefern und am Einsatzort zusammenzubauen.

Der Reaktor des erfindungsgemäßen Verfahrens wird im Folgenden anhand von Zeichnungen näher erläutert.

Es zeigen im Einzelnen:
- Figur 1: einen Längsschnitt durch eine bevorzugte Ausführungsform eines Reaktors für das erfindungsgemäße Verfahren mit Querschnitt C-C in Figur 1A sowie weitere bevorzugte Anordnungen von Thermoblechplattenmodulen im Querschnitt in den Figuren 1B bis 1F,
- Figur 2: eine Detaildarstellung aus einem Thermoblechplattenmodul im Querschnitt zu den Thermoblechplatten, mit Längsschnittdarstellungen in den Ebenen A-A bzw. B-B in Figur 2A bzw. 2B.
- Figur 3: zwei mögliche Ausführungsformen von Abdichtungen zwischen Halteboden und Stabilisierungskisten,
- Figur 4: eine Detaildarstellung mit Bohrungen in den rechteckigen Stabilisierungskästen,
- Figuren 5A, 5B 5D bis 5I: und Detaildarstellungen mit zusätzlichen Führungs- und Ausrichtungselementen an den Außenseiten der Thermoblechplattenmodule und
- Figur 6: eine Detaildarstellung einer Spanneinrichtung zur Fixierung der Thermoblechplattenmodule in den rechteckigen Stabilisierungskästen.

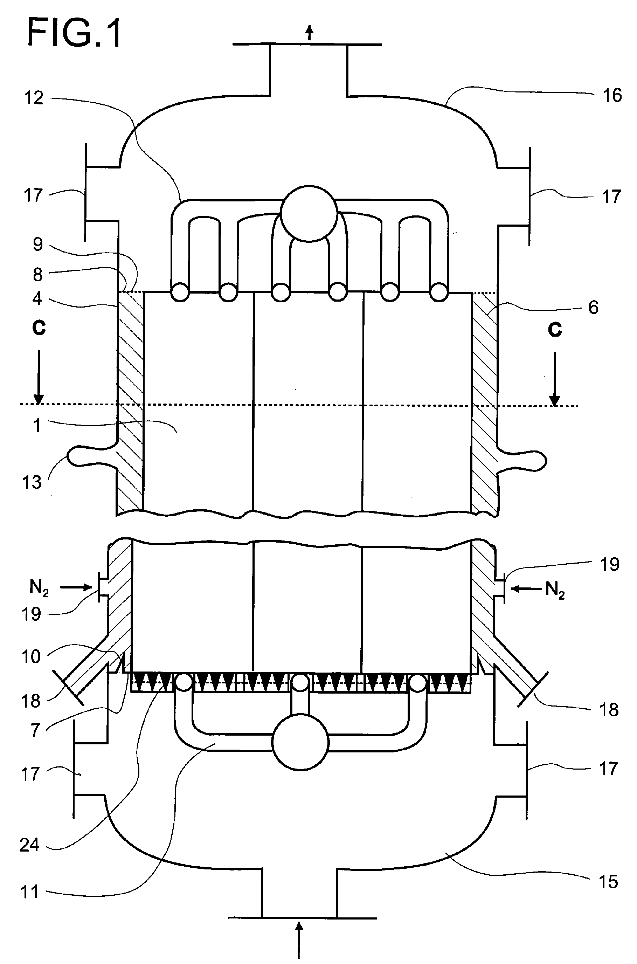

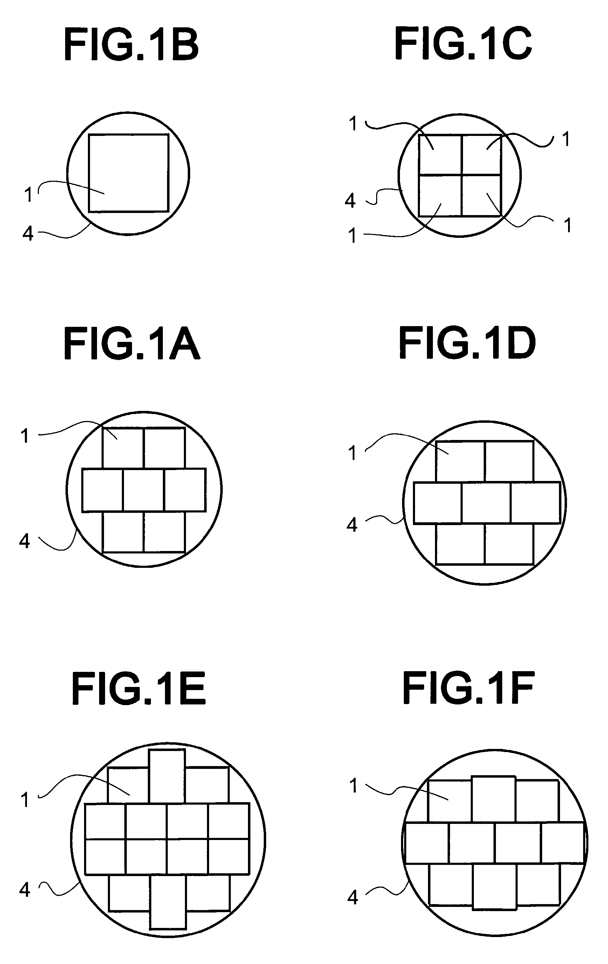

Die Längsschnittdarstellung durch eine bevorzugte Ausführungsform in Fig. 1 zeigt einen Reaktor mit Thermoblechplattenmodulen 1, die von einer überwiegend zylinderförmigen Hülle 4 umgeben sind. Der Zwischenraum 6 zwischen den Thermoblechplattenmodulen 1 und der überwiegend zylinderförmigen Hülle 4 ist im Bereich des unteren Endes der Thermoblechplattenmodule 1 durch einen Halteboden 7 gasdicht und im Bereich des oberen Endes der Thermoblechplattenmodule 1 durch eine Blechabdeckung 8, die in der Figur dargestellten bevorzugten Ausführungsform Öffnungen 9 aufweist, verschlossen.

Am unteren Ende der Thermoblechplattenmodule 1 ist eine Verteileinrichtung 11 für den Wärmeträger, häufig Speisewasser, vorgesehen und im Bereich des oberen Endes der Thermoblechplattenmodule 1 eine Sammeleinrichtung 12 für den Wärmeträger, den in diesem Bereich häufig als Dampf oder als Wasser/Dampf-Gemisch vorliegt. Die überwiegend zylinderförmige Hülle 4 weist Kompensatoren 13 für die thermische Ausdehnung auf.

In der in Fig. 1 dargestellten bevorzugten Ausführungsform wird das fluide Reaktionsmedium über die untere Haube 15 zugeführt und über die obere Haube 16 abgezogen. Im Bereich der unteren Haube 15 wie auch im Bereich der oberen Haube 16 ist jeweils ein zusätzlicher zylinderförmiger Schuss eingebaut, mit jeweils zwei Mannlöchern 17. In der überwiegend zylindrischen Hülle 4 sind Stutzen 18 für die Entleerung des Inertmaterials aus dem Zwischenraum 6 zwischen den Thermoblechmodulen 1 und der überwiegend zylinderförmigen Hülle 4 vorgesehen sowie Stutzen 19 für die Zuführung von Stickstoff in den Zwischenraum 6. Der Katalysator wird durch Katalysatorroste 24 zurückgehalten, die beispielsweise als Kantenspaltsiebe ausgebildet sind.

Die Querschnittsdarstellung in der Ebene C-C in Fig. 1A zeigt eine bevorzugte Anordnung von vorteilhaft sieben Thermoblechplattenmodulen 1 mit Zwischenraum 6 zwischen den Thermoblechplattenmodulen 1 und der Hülle 4, der bevorzugt mit Inertmaterial befüllt ist.

Figur 1B zeigt eine Querschnittsdarstellung mit einem einzigen Thermoblechplattenmodul mit quadratischem Querschnitt, das in der Hülle 4 angeordnet ist,

Figur 1C eine Ausführungsform mit vier im Querschnitt quadratischen Thermoblechplattenmodulen 1 in der Hülle 4,

Figur 1D eine Ausführungsform mit sieben im Querschnitt rechteckigen Thermoblechplattenmodulen mit dem Seitenverhältnis von jeweils 1 : 1,2,

Figur 1E eine Ausführungsform mit elf im Querschnitt rechteckigen Thermoblechplattenmodulen, mit dem Seitenverhältnis von jeweils 1 : 1,1 und

Figur 1F eine Ausführungsform mit zehn Thermoblechplattenmodulen 1 mit jeweils rechteckigem Querschnitt, mit dem Seitenverhältnis von jeweils 1 : 1,1.

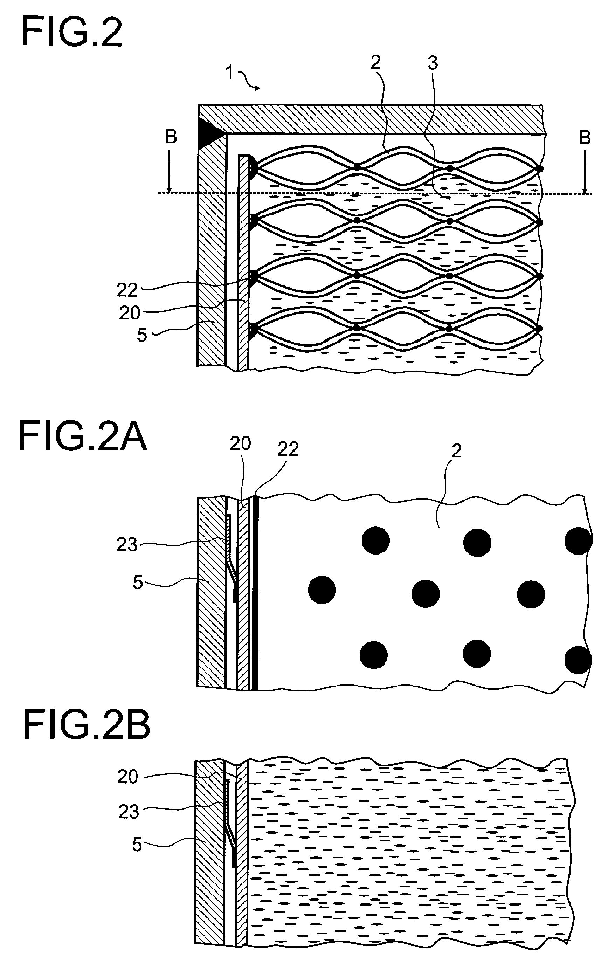

In Fig. 2 ist ein Ausschnitt aus einem Thermoblechplattenmodul 1 dargestellt, mit Thermoblechplatten 2 und Spalten 3 zwischen den Thermoblechplatten zur Aufnahme des heterogenen partikelförmigen Katalysators. In der Figur sind die Schweißpunkte zwischen den die einzelnen Thermoblechplatten 2 bildenden Bleche dargestellt sowie die Befestigung der Thermoblechplatten 2 an deren seitlichen Enden in einer seitlichen Begrenzung 20. Das Thermoblechplattenmodul ist in einen rechteckigen Stabilisierungskasten 5 eingeschoben.

In der Schnittdarstellung in der Ebene A-A in Fig. 2A ist die seitliche Rollnahtschweißung 22, die einzelne Thermoblechplatten verschließt, dargestellt sowie die Dichtstreifen 23 zwischen den Thermoblechplatten 2 des Thermoblechplattenmodules 1 und der Wand des rechteckigen Stabilisierungskasten 5. Die Figur zeigt auch eine bevorzugte Anordnung der Schweißpunkte auf den Thermoblechplatten 2.

Der Schnitt B-B, der in Fig. 2B dargestellt ist, ist in einer Ebene durch den mit dem partikelförmigen Katalysator gefüllten Spalt 3 gelegt. Zwischen der seitlichen Begrenzung 20 des Thermoblechplattenmodules 1 und der Wand des rechteckigen Stabilisierungskastens 5 sind Dichtstreifen 23 vorgesehen.

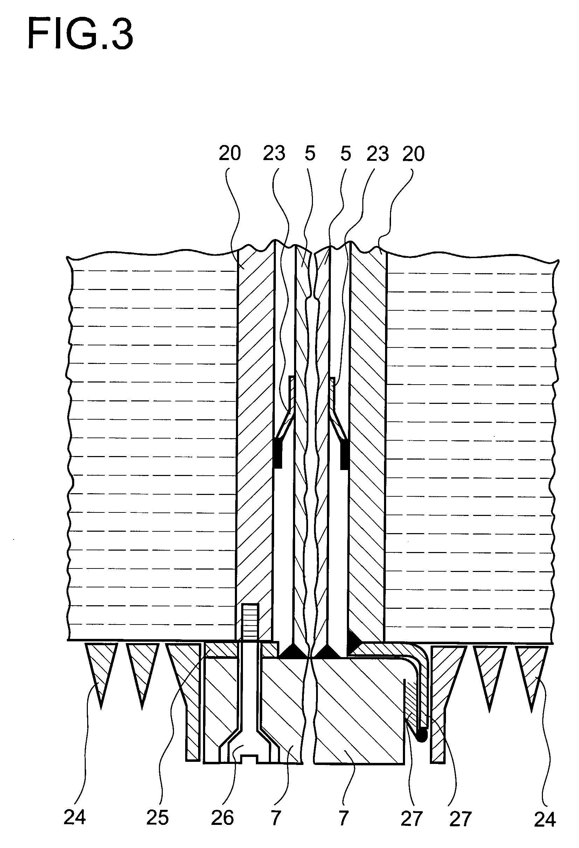

Figur 3 zeigt zwei unterschiedliche Varianten zur Abdichtung der Thermoblechplattenmodule gegen den Halteboden. Die linke Seite der Darstellung zeigt eine Dichtung 25 zwischen dem Halteboden 7 und der seitlichen Begrenzung 20 eines Thermoblechplattenmodules, wobei die Verbindung durch eine Schraube 26 fixiert ist. Der Ausschnitt zeigt auch einen Teil des als Katalysatorrost eingesetzten Kantenspaltsiebes 24 sowie einen Dichtstreifen 23 zwischen der seitlichen Begrenzung 20 des Thermoblechplattenmodules und dem rechteckigen Stabilisierungskasten 5.

Die rechte Seite der Darstellung in Figur 3 zeigt eine weitere Variante einer Abdichtung zwischen Halteboden 7 und Thermoblechplattenmodul, und zwar mittels zweier Schweißlippen 27, wovon eine am Halteboden 7 und die zweite an der seitlichen Begrenzung 20 des Thermoblechplattenmodules angeschweißt ist. Die beiden Schweißlippen sind anschließend durch eine Schweißnaht miteinander verbunden.

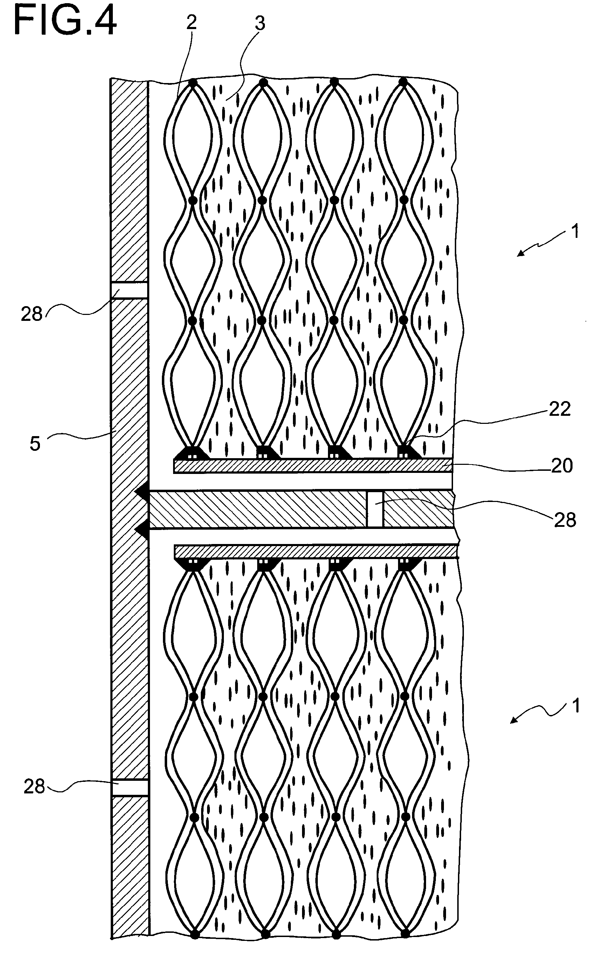

Figur 4 zeigt eine Ausführungsform mit Bohrungen 28 in den rechteckigen Stabilisierungskästen 5, wodurch zur Druckbeaufschlagung eingesetztes Gas aus dem Zwischenraum zwischen den Thermoblechplattenmodulen und der Hülle in die Räume zwischen den Thermoblechplattenmodulen 1 und den rechteckigen Stabilisierungskästen 5 einströmen kann.

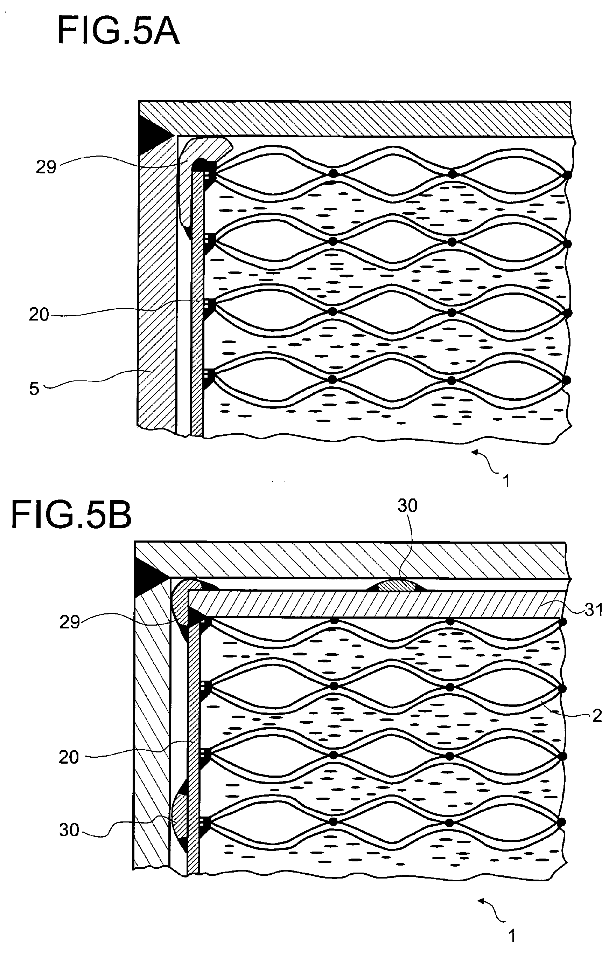

Figur 5A zeigt eine in Detaildarstellung mit einem Eckwinkel 29 an der Außenseite der seitlichen Begrenzung 20 eines Thermoblechplattenmodules 1 zur Führung und Ausrichtung gegenüber dem rechteckigen Stabilisierungskasten 5.

Die Detaildarstellung in Figur 5B zeigt zusätzlich zum Eckwinkel 29 konische Blechstreifen 30 an der Seite der Thermoblechplattenmodule 1 als Führungs- und Ausrichtungselemente.

Darüber hinaus zeigt Figur 5B eine mögliche Ausführungsform für die im Thermoblechplattenmodul 1 zu äußerst angeordnete Thermoblechplatte 2, und zwar ist das äußere Blech der äußersten Thermoblechplatte 2 des Thermoblechplattenmodules 1 dicker und damit stabiler gegenüber den übrigen, die Thermoblechplatten 2 bildenden Bleche.

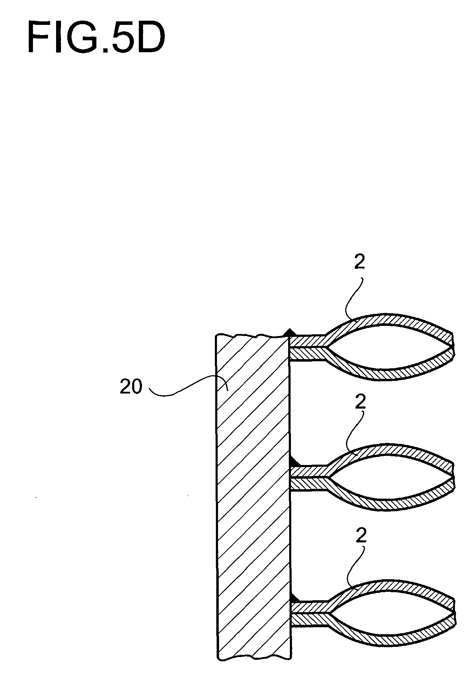

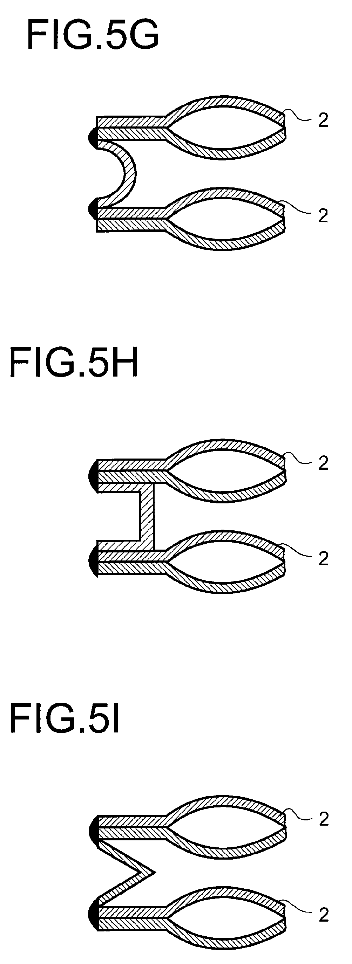

Die Figuren 5D bis 5I zeigen schematisch verschiedene Varianten zur Befestigung der Thermoblechplatten 2 an der seitlichen Begrenzung 20:
in der Ausführungsform in Figur 5D sind die Thermoblechplatten 2 angeschweißt,

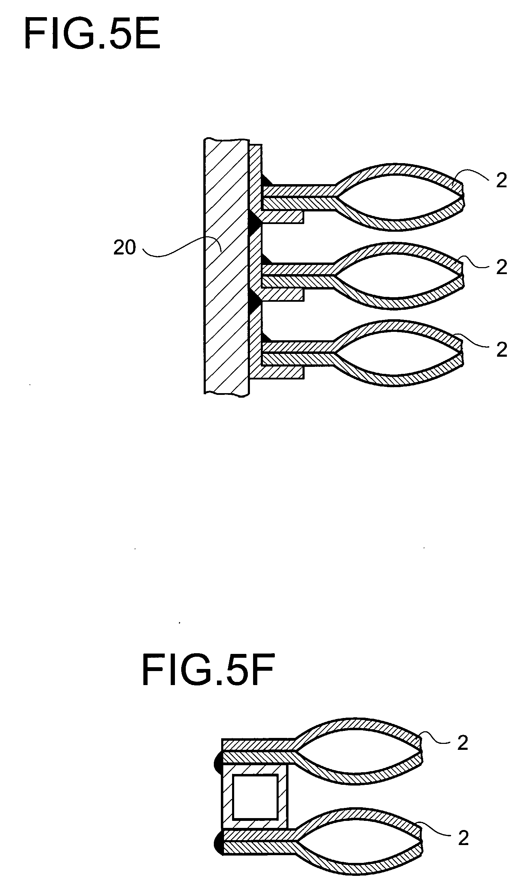

in Figur 5E sind zur Befestigung der Thermoblechplatten zwei an der seitlichen Begrenzung 20 verschweißte Winkel vorgesehen,
in der Ausführungsform in Figur 5F Vierkantrohre,
in der Ausführungsform in Figur 5G Halbrohre,
in der Ausführungsform in Figur 5H U-Profile und
in der Figur 5I Winkelprofile.

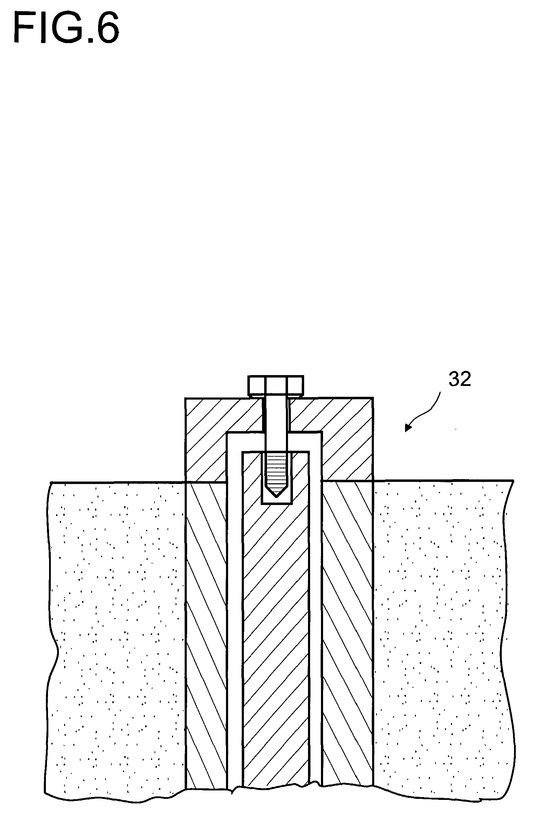

Figur 6 zeigt schematisch eine Spanneinrichtung 32 zur Verspannung zwischen Thermoblechplattenmodulen und den rechteckigen Stabilisierungskästen 5.

## Patentansprüche

1. Verfahren zur Partialoxidation in der Gasphase von C3 und/oder C4-Vorläuferverbindungen zu (Meth)acrolein und/oder (Meth)acrylsäure in Gegenwart eines heterogenen partikelförmigen Katalysators in einem Reaktor, mit
- einem oder mehreren quaderförmigen Thermoblechplattenmodulen (1), die jeweils aus zwei oder mehreren rechteckigen, parallel zueinander unter Freilassung jeweils eines Spaltes (3) angeordneten Thermoblechplatten (2) gebildet sind, der mit dem heterogenen partikelförmigen Katalysator gefüllt ist und der vom fluiden Reaktionsgemisch durchströmt wird, wobei die Reaktionswärme von einem Wärmeträger aufgenommen wird, der die Thermoblechplatten (2) durchströmt und dabei zumindest teilweise verdampft, sowie mit
- einer die Thermoblechplattenmodule (1) druckentlastenden, dieselben vollständig umgebenden überwiegend zylinderförmigen Hülle (4, 15, 16), umfassend einen Zylindermantel (4) und denselben an beiden Enden abschließenden Hauben (15, 16) und deren Längsachse parallel zur Ebene der Thermoblechplatten (2) ausgerichtet ist, sowie mit
- einem oder mehreren Abdichtelementen (7, 23), die dergestalt angeordnet sind, dass das fluide Reaktionsgemisch außer durch die von den Hauben (15, 16) begrenzten Reaktorinnenräume nur durch die Spalte (3) strömt,
- wobei der Zwischenraum (6) zwischen den Thermoblechplattenmodulen (1) und der überwiegend zylinderförmigen Hülle (4) mit einem Gasdruck beaufschlagt ist,
indem die Druckbeaufschlagung statisch ist und durch druckgeregelte Zuund Abfuhr von Stickstoff bewirkt wird oder indem
die Druckbeaufschlagung unter kontinuierlicher Durchströmung des Zwischenraumes (6) mit einem prozessinerten oder prozessintrinsischem Gas, insbesondere mit Stickstoff oder Kreisgas bewirkt wird,
- oder wobei zusätzlich zur Druckbeaufschlagung der Zwischenraum (6) zwischen den Thermoblechplattenmodulen (1) und der überwiegend zylinderförmigen Hülle (4) mit einem Inertstoff-Schüttgut aus einem chemisch inerten und mechanisch und thermisch ausreichend stabilen Material befüllt ist..

2. Verfahren nach Anspruch 1, **dadurch gekennzeichnet, dass** der Reaktor zwei oder mehrere quaderförmige Thermoblechplattenmodule (1) mit jeweils gleichen Abmessungen aufweist.

3. Verfahren nach Anspruch 2, **dadurch gekennzeichnet, dass** der Reaktor 4, 7, 10 oder 14 Thermoblechplattenmodule (1) umfasst.

4. Verfahren nach einem der Ansprüche 1 bis 3, **dadurch gekennzeichnet, dass** die Thermoblechplatten (2) aus jeweils zwei rechteckigen Blechen gebildet sind, die an ihren Längs- und Stirnseiten durch Rollnahtschweißung verbunden sind, wobei der über die Rollnaht nach außen abstehende Rand der Bleche am Außenrand der Rollnaht oder in der Rollnaht selbst abgetrennt wird.

5. Verfahren nach einem der Ansprüche 1 bis 4, **dadurch gekennzeichnet, dass** die Thermoblechplattenmodule (1) in jeweils einem druckstabilen, rechteckigen Stabilisierungskasten (5) angeordnet sind.

6. Verfahren nach Anspruch 5, **dadurch gekennzeichnet, dass** die rechteckigen Stabilisierungskästen (5) benachbarter Thermoblechplattenmodule (1) gegeneinander dichtgeschweißt sind.

7. Verfahren nach einem der Ansprüche 1 bis 6, **dadurch gekennzeichnet, dass** als Abdichtelement ein Halteboden (7) vorgesehen ist, der den Zwischenraum (6) zwischen den Thermoblechplattenmodulen (1) und der überwiegend zylinderförmigen Hülle (4) am unteren Ende der Thermoblechplattenmodule (1) verschließt.

8. Verfahren nach Anspruch 7, **gekennzeichnet durch** eine Blechabdeckung (8), die den Zwischenraum (6) zwischen den Thermoblechplattenmodulen (1) und der überwiegend zylinderförmigen Hülle (4) am oberen Ende der Thermoblechplattenmodule (1) verschließt.

9. Verfahren nach Anspruch 8, **dadurch gekennzeichnet, dass** die Blechabdeckung (8) Öffnungen (9) aufweist.

10. Verfahren nach Anspruch 1, **dadurch gekennzeichnet, dass** das Inertmaterial expandierter Perlite und/oder expandierter Vermiculit ist.

11. Verfahren nach Anspruch 1, **dadurch gekennzeichnet, dass** die Druckbeaufschlagung im Wesentlichen statisch ist.

12. Verfahren nach Anspruch 11, **dadurch gekennzeichnet, dass** die im Wesentlichen statische Druckbeaufschlagung durch druckgeregelte Zu- und Abfuhr von Stickstoff bewirkt wird.

13. Verfahren nach Anspruch 1, **dadurch gekennzeichnet, dass** die Druckbeaufschlagung durch kontinuierliche Durchströmung des Zwischenraums (6) mit einem prozessinerten oder prozessintrinsischen Gas, insbesondere mit Stickstoff oder Kreisgas, bewirkt wird.

14. Verfahren nach Anspruch 13, **dadurch gekennzeichnet, dass** das zur Druckbeaufschlagung verwendete Gas mit dem fluiden Reaktionsgemisch an dessen Austritt aus den Thermoblechplattenmodulen (1) vereinigt wird.

15. Verfahren nach einem der Ansprüche 1 bis 14, **dadurch gekennzeichnet, dass** im oder am Halteboden (7) Kompensatoren (10) für die radiale Ausdehnung vorgesehen sind.

16. Verfahren nach einem der Ansprüche 1 bis 15, **dadurch gekennzeichnet, dass** in oder an der Blechabdeckung (8) Kompensatoren (10) für die axiale und/oder radiale Ausdehnung vorgesehen sind.

17. Verfahren nach einem der Ansprüche 1 bis 16, **dadurch gekennzeichnet, dass** je Thermoblechplattenmodul ein oder mehr Verteileinrichtungen (11) und ein oder mehr Sammeleinrichtungen (12) für den Wärmeträger zum Einsatz kommen.

18. Verfahren nach einem der Ansprüche 1 bis 17, **dadurch gekennzeichnet, dass** je Thermoblechplattenmodul eine Verteileinrichtung (11) und zwei Sammeleinrichtungen (12) für den Wärmeträger zum Einsatz kommen.

19. Verfahren nach einem der Ansprüche 1 bis 18, **dadurch gekennzeichnet, dass** die Verteil- und Sammeleinrichtungen für den Wärmeträger einheitliche Abmessungen haben.

20. Verfahren nach einem der Ansprüche 1 bis 18, **dadurch gekennzeichnet, dass** die Verteileinrichtungen (11) und Sammeleinrichtungen (12) für den Wärmeträger mit Schlitzbodeneinschweißung ausgeführt werden.

21. Verfahren nach einem der Ansprüche 1 bis 18, **dadurch gekennzeichnet, dass** Verteileinrichtungen (11) und Sammeleinrichtungen (12) für den die Thermoblechplatten (2) durchströmenden Wärmeträger vorgesehen sind, die jeweils eine Kompensation für die Aufnahme der thermischen Ausdehnung der Thermoblechplattenmodule (1) relativ zur umgebenden überwiegend zylinderförmigen Hülle (4) aufweisen.

22. Verfahren nach einem der Ansprüche 1 bis 17, **dadurch gekennzeichnet, dass** die Aufnahme der thermischen Ausdehnung der Thermoblechplattenmodule (1) relativ zur umgebenden überwiegend zylinderförmigen Hülle (4) durch geeignete bogenförmige geometrische Gestaltung der Verrohrung der Verteileinrichtungen (11) und Sammeleinrichtungen (12) für den die Thermoblechplatten (2) durchströmenden Wärmeträger erfolgt.

23. Verfahren nach einem der Ansprüche 1 bis 21, **dadurch gekennzeichnet, dass** zwei Katalysatorhalteroste je Thermoblechplattenmodul zum Einsatz kommen.

24. Verfahren nach einem der Ansprüche 1 bis 22, **dadurch gekennzeichnet, dass** die Katalysatorhalteroste durch die am Reaktor vorhandenen Mannlöcher in den Reaktormantel eingebracht werden können.

25. Verfahren nach einem der Ansprüche 1 bis 23, **dadurch gekennzeichnet**, das im Zylindermantel (4) der überwiegend zylinderförmigen Hülle (4, 15, 16) ein oder mehrere Kompensatoren (13) zur Aufnahme vorzugsweise der axialen thermischen Ausdehnung vorgesehen sind.

26. Verfahren nach einem der Ansprüche 1 bis 24, wobei die Reaktionswärme durch einen die Thermoblechplatten (2) durchströmenden Wärmeträger abgeführt wird, der hierbei zumindest teilweise verdampft.

27. Verfahren nach Anspruch 25, **dadurch gekennzeichnet, dass** die Aufheizung der Thermoblechplattenmodule (1) zur Inbetriebnahme des Reaktors aus demselben Wärmeträgernetz erfolgt, in das beim Reaktionsbetrieb die Wärme durch zumindest teilverdampftes Wärmeträgermedium abgeführt wird.

## Claims

1. A process for partial oxidation in the gas phase of C3 and/or C4 precursor compounds to (meth)acrolein and/or (meth)acrylic acid in the presence of a heterogeneous particulate catalyst in a reactor having
- one or more cuboidal thermoplate modules (1) which are each formed from two or more rectangular thermoplates (2) arranged parallel to each other while in each case leaving a gap (3) which is filled with the heterogeneous particulate catalyst and is flowed through by the fluid reaction mixture, the heat of reaction being absorbed by a heat carrier which flows through the thermoplates (2) and thus at least partly evaporating, and also having
- a predominantly cylindrical shell (4, 15, 16) which releases the pressure at the thermoplate modules (1), completely surrounds them and comprises a cylinder jacket (4) and hoods (15, 16) which seal it at both ends and whose longitudinal axis is aligned parallel to the plane of the thermoplates (2), and also having
- one or more sealing elements (7, 23) which are arranged in such a way that the fluid reaction mixture, apart from flowing through the reactor interior spaces bounded by the hoods (15, 16), only flows through the gap (3),
- wherein a gas pressure is applied to the intermediate space (6) between the thermoplate modules (1) and the predominantly cylindrical shell (4), in that the application of pressure is constant and is effected by pressure-regulated feed and removal of nitrogen or in that the pressure is applied with continuous feeding of a gas which is inert or intrinsic to the process, particularly of nitrogen or cycle gas, through the intermediate space (6),
- or wherein, in addition to the application of pressure, the intermediate space (6) between the thermoplate modules (1) and the predominantly cylindrical shell (4) is filled with an inert bed material consisting of a chemically inert and sufficiently mechanically and thermally stable material.

2. The process according to claim 1, wherein the reactor has two or more cuboidal thermoplate modules (1) each having the same dimensions.

3. The process according to claim 2, wherein the reactor comprises 4, 7, 10 or 14 thermoplate modules (1).

4. The process according to any of claims 1 to 3, wherein the thermoplates (2) are formed from in each case two rectangular metal sheets which are joined at their longitudinal and end sides by roll seam welding, and the edge of the metal sheets protruding outward over the roll seam is removed at the outer edge of the roll seam or in the roll seam itself.

5. The process according to any of claims 1 to 4, wherein the thermoplate modules (1) are in each case arranged in a pressure-stable rectangular stabilization frame (5).

6. The process according to claim 5, wherein the rectangular stabilization frames (5) of adjacent thermoplate modules (1) are welded and sealed together.

7. The process according to any of claims 1 to 6, wherein the sealing element provided is a holding base (7) which seals the intermediate space (6) between the thermoplate modules (1) and the predominantly cylindrical shell (4) at the lower ends of the thermoplate modules (1).

8. The process according to claim 7, wherein a metal metal sheet cover (8) seals the intermediate space (6) between the thermoplate modules (1) and the predominantly cylindrical shell (4) at the upper end of the thermoplate modules (1).

9. The process according to claim 8, wherein the metal metal sheet cover (8) has orifices (9).

10. The process according to claim 1, wherein the inert material is expanded perlite and/or expanded vermiculite.

11. The process according to claim 1, wherein the pressure applied is substantially constant.

12. The process according to claim 11, wherein the substantially constant application of pressure is effected by pressure-regulated feed and removal of nitrogen.

13. The process according to claim 1, wherein the pressure is applied by continuously feeding a gas which is inert or intrinsic to the process, in particular nitrogen or cycle gas, through the intermediate space (6).

14. The process according to claim 13, wherein the gas used to apply pressure is combined with the fluid reaction mixture at its outlet from the thermoplate modules (1).

15. The process according to any of claims 1 to 14, wherein compensators (10) for the radial expansion are provided in or on the holding base (7).

16. The process according to any of claims 1 to 15, wherein compensators (10) for the axial and/or radial expansion are provided in or on the metal metal sheet cover (8).

17. The process according to any of claims 1 to 16, wherein one or more distribution devices (11) and one or more collection devices (12) for the heat carrier are used per thermoplate module.

18. The process according to any of claims 1 to 17, wherein one distribution device (11) and two collection devices (12) for the heat carrier are used per thermoplate module.

19. The process according to any of claims 1 to 18, wherein the distribution and collection devices for the heat carrier have uniform dimensions.

20. The process according to any of claims 1 to 18, wherein the distribution devices (11) and collection devices (12) for the heat carrier are welded into a slotted base.

21. The process according to any of claims 1 to 18, wherein distribution devices (11) and collection devices (12) for the heat carrier flowing through the thermoplates (2) are provided which each have compensation for the accommodation of the thermal expansion of the thermoplate modules (1) relative to the surrounding, predominantly cylindrical shell (4).

22. The process according to any of claims 1 to 17, wherein the thermal expansion of the thermoplate modules (1) relative to the surrounding, predominantly cylindrical shell (4) is accommodated by suitable curved geometric design of the tubing of the distribution devices (11) and collection devices (12) for the heat carrier flowing through the thermoplates (2).

23. The process according to any of claims 1 to 21, wherein two catalyst holding grates per thermoplate module are used.

24. The process according to any of claims 1 to 22, wherein the catalyst holding grates can be introduced into the reactor jacket through the manholes present in the reactor.

25. The process according to any of claims 1 to 23, wherein one or more compensators (13) for accommodating preferably the axial thermal expansion are provided in the cylinder jacket (4) of the predominantly cylindrical shell (4, 15, 16).

26. The process according to any of claims 1 to 24, wherein the heat of reaction is removed by a heat carrier flowing through the thermoplates (2) which is thus at least partly evaporated.

27. The process according to claim 25, wherein the thermoplate modules (1) for starting up the reactor are heated by the same heat carrier network into which the heat is removed in the course of reaction operation by at least partially evaporated heat carrier medium.

## Revendications

1. Procédé pour l'oxydation partielle en phase gazeuse de composés précurseurs en C3 et/ou en C4 en (méth)acroléine et/ou en acide (méth)acrylique en présence d'un catalyseur hétérogène sous forme de particules dans un réacteur présentant
- un ou plusieurs modules (1) à plaques en tôle thermique parallélépipédiques, qui sont formés à chaque fois à partir de deux plaques (2) en tôle thermique ou plus, rectangulaires, disposées parallèlement les unes aux autres en laissant à chaque fois une fente (3), qui est remplie par le catalyseur hétérogène sous forme de particules et qui est traversée par un mélange réactionnel fluide, la chaleur de réaction étant absorbée par un caloporteur qui s'écoule au travers des plaques (2) en tôle thermique et qui s'évapore ainsi au moins partiellement ainsi que
- une gaine (4, 15, 16) principalement cylindrique entourant complètement et éliminant la pression des modules (1) à plaques en tôle thermique, comprenant une enveloppe cylindrique (4) et des capots (15, 16) qui la ferment aux deux extrémités et dont l'axe longitudinal est orienté parallèlement au plan des plaques (2) en tôle thermique, ainsi que
- un ou plusieurs éléments d'étanchéité (7, 23), qui sont disposés de manière telle que le mélange réactionnel fluide, en plus de s'écouler à travers les espaces internes du réacteur délimités par les capots (15, 16), s'écoule uniquement à travers les fentes (3),
- l'espace intermédiaire (6) entre les modules (1) à plaques en tôle thermique et la gaine (4) principalement cylindrique étant sollicité en pression gazeuse en ce que la sollicitation en pression est statique et est provoquée par l'alimentation et l'évacuation régulées en pression d'azote ou en ce que la sollicitation en pression est provoquée par écoulement continu à travers l'espace intermédiaire (6) d'un gaz inerte ou intrinsèque au procédé, en particulier de l'azote ou un gaz en circulation,
- ou l'espace intermédiaire (6) entre les modules (1) à plaques en tôle thermique et la gaine (4) principalement cylindrique, en plus de la sollicitation en pression, étant rempli par un matériau inerte en vrac constitué par un matériau chimiquement inerte et mécaniquement et thermiquement suffisamment stable.

2. Procédé selon la revendication 1, **caractérisé en ce que** le réacteur présente deux modules (1) à plaques en tôle thermique parallélépipédiques ou plus, présentant à chaque fois des dimensions identiques.

3. Procédé selon la revendication 2, **caractérisé en ce que** le réacteur comprend 4, 7, 10 ou 14 modules (1) à plaques en tôle thermique.

4. Procédé selon l'une quelconque des revendications 1 à 3, **caractérisé en ce que** les plaques (2) en tôle thermique sont formées par à chaque fois deux tôles rectangulaires, qui sont reliées au niveau de leurs côtés longitudinaux et frontaux par soudage à la molette, le bord éloigné vers l'extérieur au-delà du joint de la tôle étant séparé au niveau du bord extérieur du joint ou dans le joint.

5. Procédé selon l'une quelconque des revendications 1 à 4, **caractérisé en ce que** les modules (1) à plaques en tôle thermique sont disposés dans à chaque fois une boîte de stabilisation (5) stable à la pression, rectangulaire.

6. Procédé selon la revendication 5, **caractérisé en ce que** les modules (1) à plaques en tôle thermique adjacents aux boîtes de stabilisation (5) rectangulaires sont soudés de manière étanche l'un contre l'autre.

7. Procédé selon l'une quelconque des revendications 1 à 6, **caractérisé en ce que** l'élément d'étanchéité est un fond de retenue (7) qui ferme l'espace intermédiaire (6) entre les modules (1) à plaques en tôle thermique et la gaine (4) principalement cylindrique au niveau de l'extrémité inférieure du module (1) à plaques en tôle thermique.

8. Procédé selon la revendication 7, **caractérisé par** un recouvrement (8) en tôle qui ferme l'espace intermédiaire (6) entre les modules (1) à plaques en tôle thermique et la gaine (4) principalement cylindrique au niveau de l'extrémité supérieure des modules (1) à plaques en tôle thermique.

9. Procédé selon la revendication 8, **caractérisé en ce que** le recouvrement (8) en tôle présente des ouvertures (9).

10. Procédé selon la revendication 1, **caractérisé en ce que** le matériau inerte est de la perlite expansée et/ou de la vermiculite expansée.

11. Procédé selon la revendication 1, **caractérisé en ce que** la sollicitation en pression est essentiellement statique.

12. Procédé selon la revendication 11, **caractérisé en ce que** la sollicitation essentiellement statique en pression est provoquée par une alimentation et une évacuation régulées en pression d'azote.

13. Procédé selon la revendication 1, **caractérisé en ce que** la sollicitation en pression est provoquée par écoulement continu à travers l'espace intermédiaire (6) d'un gaz inerte ou intrinsèque au procédé, en particulier de l'azote ou un gaz en circulation.

14. Procédé selon la revendication 13, **caractérisé en ce que** le gaz utilisé pour la sollicitation en pression est rassemblé avec le mélange réactionnel fluide à sa sortie des modules (1) à plaques en tôle thermique.

15. Procédé selon l'une quelconque des revendications 1 à 14, **caractérisé en ce que** des compensateurs (10) pour la dilatation radiale sont disposés dans ou au niveau du fond de retenue (7).

16. Procédé selon l'une quelconque des revendications 1 à 15, **caractérisé en ce que** des compensateurs (10) pour la dilatation axiale et/ou radiale sont disposés dans ou au niveau du recouvrement en tôle (8).

17. Procédé selon l'une quelconque des revendications 1 à 16, **caractérisé en ce qu'**un ou plusieurs dispositifs de répartition (11) et un ou plusieurs dispositifs de collecte (12) pour le caloporteur sont utilisés par module à plaques en tôle thermique.

18. Procédé selon l'une quelconque des revendications 1 à 17, **caractérisé en ce qu'**un dispositif de répartition (11) et deux dispositifs de collecte (12) pour le caloporteur sont utilisés par module à plaques en tôle thermique.

19. Procédé selon l'une quelconque des revendications 1 à 18, **caractérisé en ce que** les dispositifs de répartition et de collecte pour le caloporteur présentent des dimensions uniformes.

20. Procédé selon l'une quelconque des revendications 1 à 18, **caractérisé en ce que** les dispositifs de répartition (11) et les dispositifs de collecte (12) pour le caloporteur sont réalisés avec incorporation par soudure d'un fond perforé.

21. Procédé selon l'une quelconque des revendications 1 à 18, **caractérisé en ce que** des dispositifs de répartition (11) et des dispositifs de collecte (12) pour le caloporteur s'écoulant à travers les plaques (2) en tôle thermique sont utilisés, qui présentent à chaque fois une compensation pour absorber la dilatation thermique des modules (1) à plaques en tôle thermique par rapport à la gaine (4) principalement cylindrique périphérique.

22. Procédé selon l'une quelconque des revendications 1 à 17, **caractérisé en ce que** l'absorption de la dilatation thermique des modules (1) à plaques en tôle thermique par rapport à la gaine (4) principalement cylindrique périphérique est réalisée par une exécution géométrique appropriée en forme d'arc de la tuyauterie des dispositifs de répartition (11) et des dispositifs de collecte (12) pour le caloporteur s'écoulant à travers les plaques (2) en tôle thermique.

23. Procédé selon l'une quelconque des revendications 1 à 21, **caractérisé en ce qu'**on utilise deux grilles de retenue de catalyseur par module à plaques en tôle thermique.

24. Procédé selon l'une quelconque des revendications 1 à 22, **caractérisé en ce que** les grilles de retenue de catalyseur peuvent être introduites dans l'enveloppe du réacteur via des trous d'homme se trouvant au niveau du réacteur.

25. Procédé selon l'une quelconque des revendications 1 à 23, **caractérisé en ce qu'**un ou plusieurs compensateurs (13) pour absorber la dilatation thermique de préférence axiale sont disposés dans la gaine (4) cylindrique de l'enveloppe (4, 15, 16) principalement cylindrique.

26. Utilisation selon l'une quelconque des revendications 1 à 24, la chaleur de réaction étant évacuée par un caloporteur, qui s'évapore au moins partiellement, s'écoulant à travers les plaques (2) en tôle thermique.

27. Procédé selon la revendication 25, **caractérisé en ce que** le chauffage des modules (1) à plaques en tôle thermique pour la mise en service du réacteur est réalisé à partir du même réseau de caloporteur dans lequel, lors du fonctionnement de la réaction, la chaleur est évacuée par le milieu caloporteur au moins partiellement évaporé.
